# EUROPEAN PATENT SPECIFICATION

(11) **EP 1 155 014 B1**
(45) Date of publication and mention of the grant of the patent: **04.06.2003**
(21) Application number: 00902282.3
(22) Date of filing: 24.01.2000
(51) Int. Cl.: C07D 403/12, C07D 417/12, A61K 31/505

(54) **NEW BENZIMIDAZOLE COMPOUNDS FOR USE AS ACTIVE THERAPEUTIC SUBSTANCES, PROCESS FOR THE PREPARATION OF THESE COMPOUDNS AND PHARMACEUTICAL COMPOSITIONS COMPRISING THEM**
BENZIMIDAZOLVERBINDUNGEN ZUR ANWENDUNG ALS THERAPEUTISCH WIRKSAME SUBSTANZEN, VERFAHREN ZUR HERSTELLUNG DIESER VERBINDUNGEN UND PHARMAZEUTISCHE ZUSAMMENSETZUNGEN DIE DIESE ENTHALTEN
NOUVEAUX COMPOSES DE BENZIMIDAZOLE POUVANT ETRE UTILISES COMME PRINCIPES ACTIFS THERAPEUTIQUES, PROCEDE PERMETTANT DE LES PREPARER ET COMPOSITIONS PHARMACEUTIQUES LES CONTENANT

(30) Priority: 25.01.1999 SE 9900211
(43) Date of publication of application: 21.11.2001
(73) Proprietor: Pharmacia AB, 112 87 Stockholm (SE)
(72) Inventor: HEDGECOCK, Charles, S-757 56 Uppsala (SE); DESARBRE, Eric, S-112 46 Stockholm (SE); KURZ, Guido, S-117 39 Stockholm (SE); NORIN, Martin, S-167 51 Bromma (SE); LUTHMAN, Marguerite, S-181 35 Lidingö (SE); WIDERST HL, Cathrin, S-167 66 Bromma (SE)
(74) Representative: Ebbinghaus, Marie-Louise
(86) International application number: SE0000144
(87) International publication number: WO00043387

(56) References cited:
- S.P. SINGH ET AL.: 'Synthesis & Mass Spectra of Some Substituted' INDIAN JOURNAL OF CHEMISTRY vol. 22B, January 1983, pages 37 - 42, XP002927472

## Description

The present invention relates to new benzimidazole compounds with the following general structure (I): 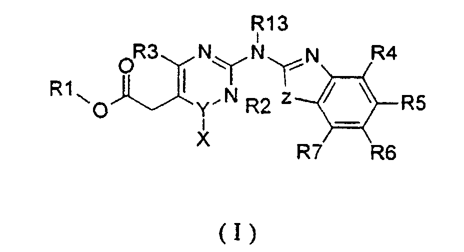 in which
Y -X is >C = X when X is NR8, O, or S and R2 is H, a straight or branched chain with 1 to 8 carbon atoms; a straight or branched chain substituted with a ring having 5-12 carbon atoms and optionally having one or two hetero atoms, a ring having 5-12 carbon atoms and optionally having one or two hetero atoms substituted with halogen, -OH, a straight or branched chain with 1 to 8 carbon atoms, OR10, NR10, R11, NO2, CF3, CN, COR8 and/or COOR8; or
Y-X is >C-X when X is H, a straight or branched chain with 1 to 8 carbon atoms, a straight or branched chain with 1 to 8 carbon atoms substituted with halogen, -OH, a straight or branched chain with 1 to 8 carbon atoms; OR10, NR10, R11, NO2, CF3, CN, COR8 and/or COOR8, a ring having 5-12 carbon atoms and optionally having one or two hetero atoms, a ring having 5-12 carbon atoms and optionally having one or two hetero atoms substituted with halogen, -OH, a straight or branched chain with 1 to 8 carbon atoms, OR10, NR10, R11, NO2, CF3, CN, COR8 and/or COOR8, or X is OR9 or NHR9 and R2 is a bond to Y;
Z = O, S or NR12;
R1 is H, methyl, propyl, butyl or isoforms thereof, a straight or branched chain with 1 to 8 carbon atoms substituted with halogen, -OH, a straight or branched chain with 1 to 8 carbon atoms OR10, NR10, R11, NO2, CF3, CN, COR8 and/or COOR8, a ring having 5-12 carbon atoms and optionally having one or two hetero atoms or a ring having 5-12 carbon atoms and optionally having one or two hetero atoms or a ring having 5-12 carbon atoms and optionally having one or two hetero atoms substituted with halogen, -OH, a straight or branched chain with 1 to 8 carbon atoms; OR10, NR10, R11, NO2, CF3, CN, COR8 and/or COOR8;
R3 is H, a straight or branched chain with 1 to 8 carbon atoms, a straight or branched chain with 1 to 8 carbon atoms substituted with halogen, -OH, a straight or branched chain with 1 to 8 carbon atoms; OR10, NR10, R11, NO2, CF3, CN, COR8 and/or COOR8, a ring having 5-12 carbon atoms and optionally having one or two hetero atoms or a ring having 5-12 carbon atoms and optionally having one or two hetero atoms substituted with halogen, -OH, a straight or branched chain with 1 to 8 carbon atoms, OR10, NR10, R11, NO2, CF3, CN, COR8 and/or COOR8, or R² is OR9 or NHR9;
R4, R5, R6, R7 are H, halogen, OR10, NR10, R11, NO2, CF3, CN, COR8, COOR8, CONHR8 and/or N3 in any combination and/or two adjacent R4, R5, R6 or R7 form a carbocyclic or heterocyclic ring;
R8, R9, R10, R11, R12 and R13 are H, a straight or branched chain with 1 to 8 carbon atoms, a straight or branched chain substituted with a ring having 5-12 carbon atoms and optionally having one or two hetero atoms, a ring having 5-12 carbon atoms and optionally having one or two hetero atoms and/or a ring having 5-12 carbon atoms and optionally having one or two hetero atoms substituted halogen, -OH, a straight or branched chain with 1 to 8 carbon atoms, OR10, NR10, R11, NO2, CF3, CN, COR8 and/or COOR8 with in any combination.

The compounds included in formula I are useful as a human Growth Hormone (hGH) mimetic, which trigger GH agonist effects in animals and especially as an orally available human Growth Hormone (hGH).

### Introduction

hGH is a protein consisting of a single chain of 191 amino acids. The molecule is crosslinked by two disulphide bridges and the monomeric form has a molecular weight of 22 kDa. Recombinant hGH (22 kDa) has been commercially available for several years. It is preferred over the pituitary derived products because the product prepared from human tissue might contain infectious agents such as that for the Creutzfeldt-Jacob's disease. Two types of therapeutically useful recombinant hGH preparations are present on the market: the natural sequence, e.g. Genotropin®. Kabi Pharmacia AB. and an analogue with an additional methionine residue at the N-terminal end. e.g. Somatonorm®.

hGH is used to stimulate linear growth in patients with hypopituitary dwarfism or Turner's syndrome but other indications have also been suggested.

The protein hGH must be administered by injection and there is a need for an oral available compound with the same biological activity as the natural occuring GH. A benzimidazoles of general formula I wherein R1 is C₂H₅, Y-X is >C -OH, Z=N and R2, R3, R4, R5, R6, R7 and R13 are H is known to have antiinflammatory activity from Singh et al, Indian J of Chemistry. Vol 22B, January 1983, 37-42. (Compound IId, IIp and IIu in Table 1).

### The invention

We have unexpectedly found a group of compounds having the biological activity of growth hormone and which could be useful as alternatives to recombinant and native growth hormone.

A number of compounds have been identified, e.g. 2-[(5,6-Dimethyl-2-benzoimidazolyl)amino]-4-hydroxy-6-methyl-5-pyrimidine acetic acid (2) and 2-[(5,6-Dimethyl-2-benzoimidazolyl)amino]-4-1-hydroxy-6-methyl-5-pyrimidine acetic acid, ethyl ester (1). (1) is found to be a selective agonist for the growth hormone receptor against the closely related prolactin receptor, which is of importance when looking for a specific growth hormone mimetic.

The invention thus relates to compounds with the general structure (1): 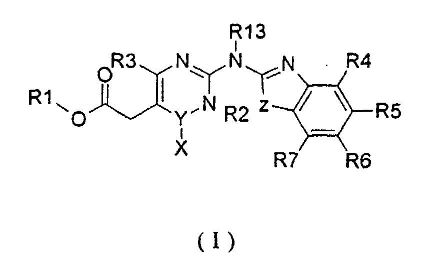 in which
Y-X is >C = X when X is NR8, O, or S and R2 is H, a straight or branched chain with 1 to 8 carbon atoms; a straight or branched chain substituted with a ring having 5-12 carbon atoms and optionally having one or two hetero atoms, a ring having 5-12 carbon atoms and optionally having one or two hetero atoms substituted with halogen, -OH, a straight or branched chain with 1 to 8 carbon atoms; OR10, NR10, R11, NO2, CF3, CN, COR8 and/or COOR8; or
Y-X is >C-X when X is H, a straight or branched chain with 1 to 8 carbon atoms, a straight or branched chain with 1 to 8 carbon atoms substituted with halogen, -OH, a straight or branched chain with 1 to 8 carbon atoms; OR10, NR10, R11, NO2, CF3, CN, COR8 and/or COOR8, a ring having 5-12 carbon atoms and optionally having one or two hetero atoms, a ring having 5-12 carbon atoms and optionally having one or two hetero atoms substituted with halogen, -OH, a straight or branched chain with 1 to 8 carbon atoms; OR10, NR10, R11, NO2, CF3, CN, COR8 and/or COOR8, OR9 or NHR9 and R2 is a bond to Y;
Z = O, S or NR12;
R1 is H, methyl, propyl, butyl or isoforms thereof, a straight or branched chain with 1 to 8 carbon atoms substituted with halogen, -OH, a straight or branched chain with 1 to 8 carbon atoms; OR10, NR10, R11, NO2, CF3, CN, COR8 and/or COOR8, a ring having 5-12 carbon atoms and optionally having one or two hetero atoms or a ring having 5-12 carbon atoms and optionally having one or two hetero atoms or a ring having 5-12 carbon atoms and optionally having one or two hetero atoms substituted with halogen, -OH, a straight or branched chain with 1 to 8 carbon atoms; OR10, NR10, R11, NO2, CF3, CN, COR8 and/or COOR8;
R3 is H, a straight or branched chain with 1 to 8 carbon atoms, a straight or branched chain with 1 to 8 carbon atoms substituted with halogen, -OH, a straight or branched chain with 1 to 8 carbon atoms; OR10, NR10, R11, NO2, CF3, CN, COR8 and/or COOR8, a ring having 5-12 carbon atoms and optionally having one or two hetero atoms or a ring having 5-12 carbon atoms and optionally having one or two hetero atoms substituted with halogen, -OH, a straight or branched chain with 1 to 8 carbon atoms; OR10, NR10, R11, NO2, CF3, CN, COR8 and/or COOR8, OR9 or NHR9;
R4, R5, R6, R7 are H, halogen, OR10, NR10, R11, NO2, CF3, CN, COR8, COOR8, CONHR8 and/or N3 in any combination and/or two adjacent R4, R5, R6 or R7 form a carbocycloc or heterocyclic ring;
R8, R9, R10, R11, R12 and R13 are H, a straight or branched chain with 1 to 8 carbon atoms, a straight or branched chain substituted with a ring having 5-12 carbon atoms and optionally having one or two hetero atoms, a ring having 5-12 carbon atoms and optionally having one or two hetero atoms and/or a ring having 5-12 carbon atoms and optionally having one or two hetero atoms substituted halogen, -OH, a straight or branched chain with 1 to 8 carbon atoms; OR10, NR10, R11, NO2, CF3, CN, COR8 and/or COOR8 with in any combination.

A straight or branched chain with 1 to 8 carbon atoms is preferably 1 to 4 carbon atoms, e.g. methyl, ethyl, propyl, butyl and isoforms thereof.

A ring having 5-12 carbon atoms, is e.g. cyclopropyl, phenyl, cyclohexyl and possibly one or two hetero atoms.

By "hetero atom" is preferably meant O, S and/or N.

The compounds could be a pharmaceutical acceptable salts and esters thereof.

More particular the following compounds are of interest:
2-[(5,6-Dimethyl-2-benzoimidazolyl)amino]-4-hydoxy-6-methyl-5-pyrimidine acetic acid, ethyl ester (1) and
2-[(5,6-DimethyI-2-benzoimidazolyl)amino]-4-hydroxy-6-methyl-5-pyrimidine acetic acid (2).

The invention also relates to use of the claims compounds, pharmaceutical compositions comprising the compounds and method for the preparation of the compounds as claimed.

We have shown biological activity for the claimed compounds by different assays, such as a dimerisation assay in which the compounds which mimics the quench induced upon hGH binding in this assay also may mimic the full biological effects of native hormone and increased glucose transport.

The claimed compounds can be useful for treatment of diseases related to e.g. growth hormone deficiency, Turner syndrome, infertility, wound healing, dystrophy, osteoporosis, and/or lactation failure, for preoperative administration and other diseases and conditions which are treated or could be treated by administration of hGH.

### Figures:

- Figure 1 A and 1B: Schemes I and II, respectively

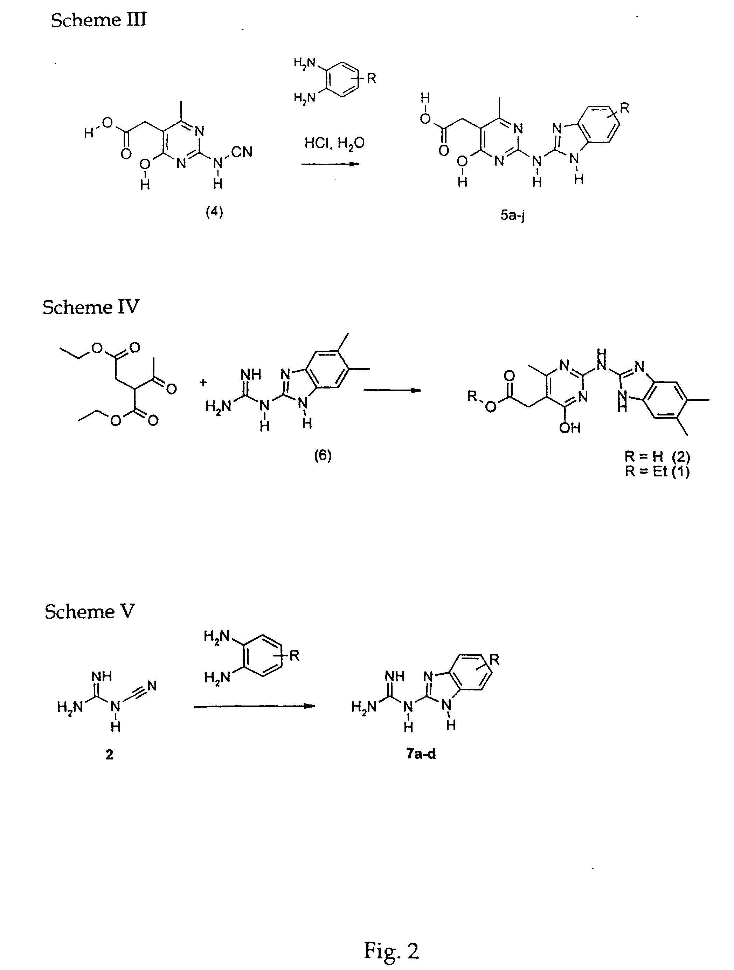

- Figure 2: Schemes III, IV and V
- Figure 3: Dose response curves of hGH and compound (2) in dimerisation assay
- Figure 4A.13: Glucose uptake in cardiac myocytes treated with medium (control), hGH (x nM) and compound (2) (10 mM)

### PREPARATION METHODS

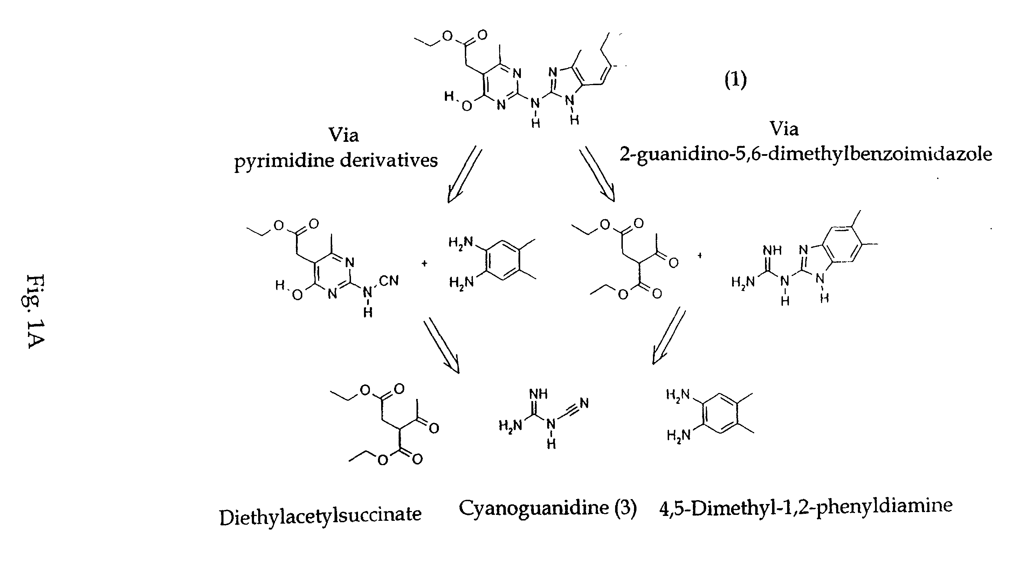

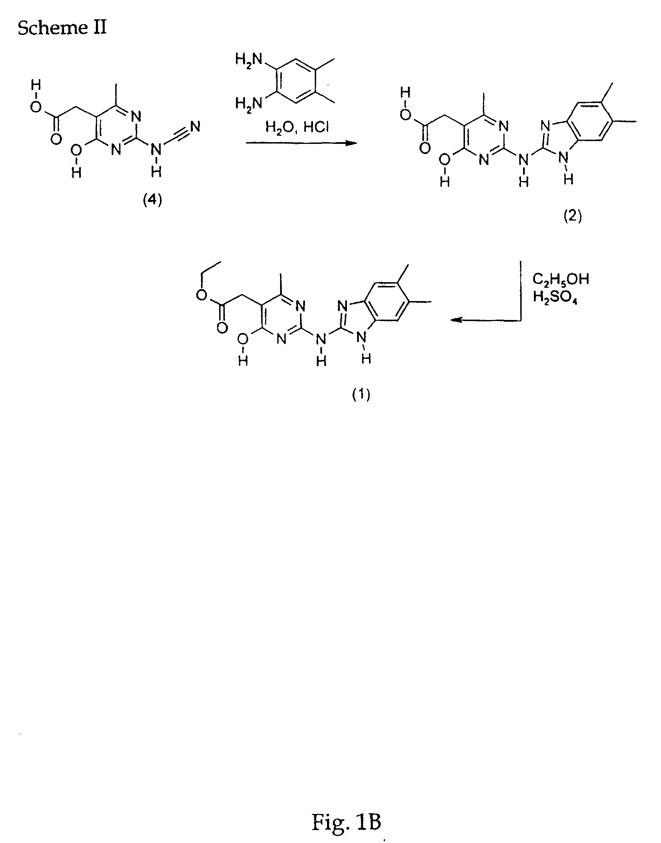

These compounds can be prepared according to two different routes :
1) via pyrimidine derivatives or
2) via the 2-guanidino-5,6-dimethylbenzoimidazole
See Figure 1, scheme I.

### Preparation of 2-[(5,6-Dimethyl-2-benzoimidazolyl)amino]-4-hydroxy-6-methyl-5-pyrimidine acetic acid, ethyl ester (1) and Analogues

### 1) Via pyrimidine derivatives

(2-Cyanoamino)-4-hydroxy-6-methyl-5-pyrimidine acetic acid (4) obtained from diethyl acetylsuccinate and cyanoguanidine in presence of sodium in ethanol, was treated with phenyldiamine in H₂O and concentrated HCl to afford 2-[(5,6-Dimethyl-2-benzoimidazolyl)amino]-4-hydroxy-6-methyl-5-pyrimidine acetic acid (2) in 25% yield. Recrystallisation of acid from DMF gave the pure compound. Esterification was performed in ethanol in presence of H₂SO₄ to lead to 2-[(5,6-Dimethyl-2-benzoimidazolyl)amino]-4-hydroxy-6-methyl-5-pyrimidine acetic acid, ethyl ester (1) in only 19 % yield (scheme II).

Synthesis of analogues 5a-j was carried out similarly by reacting (4) with different *ortho*-diamino derivatives, under the conditions used to prepare (2), as shown in scheme III.

The following compounds were prepared according to Scheme II or III:

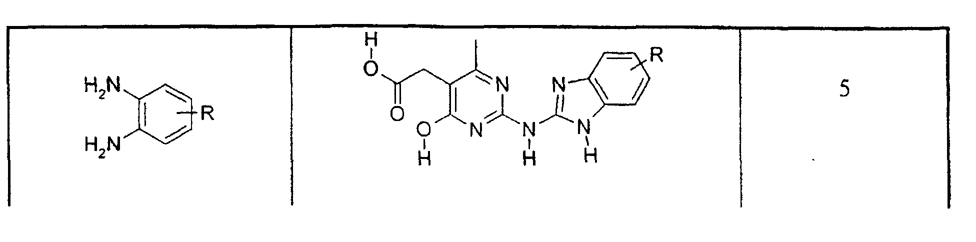

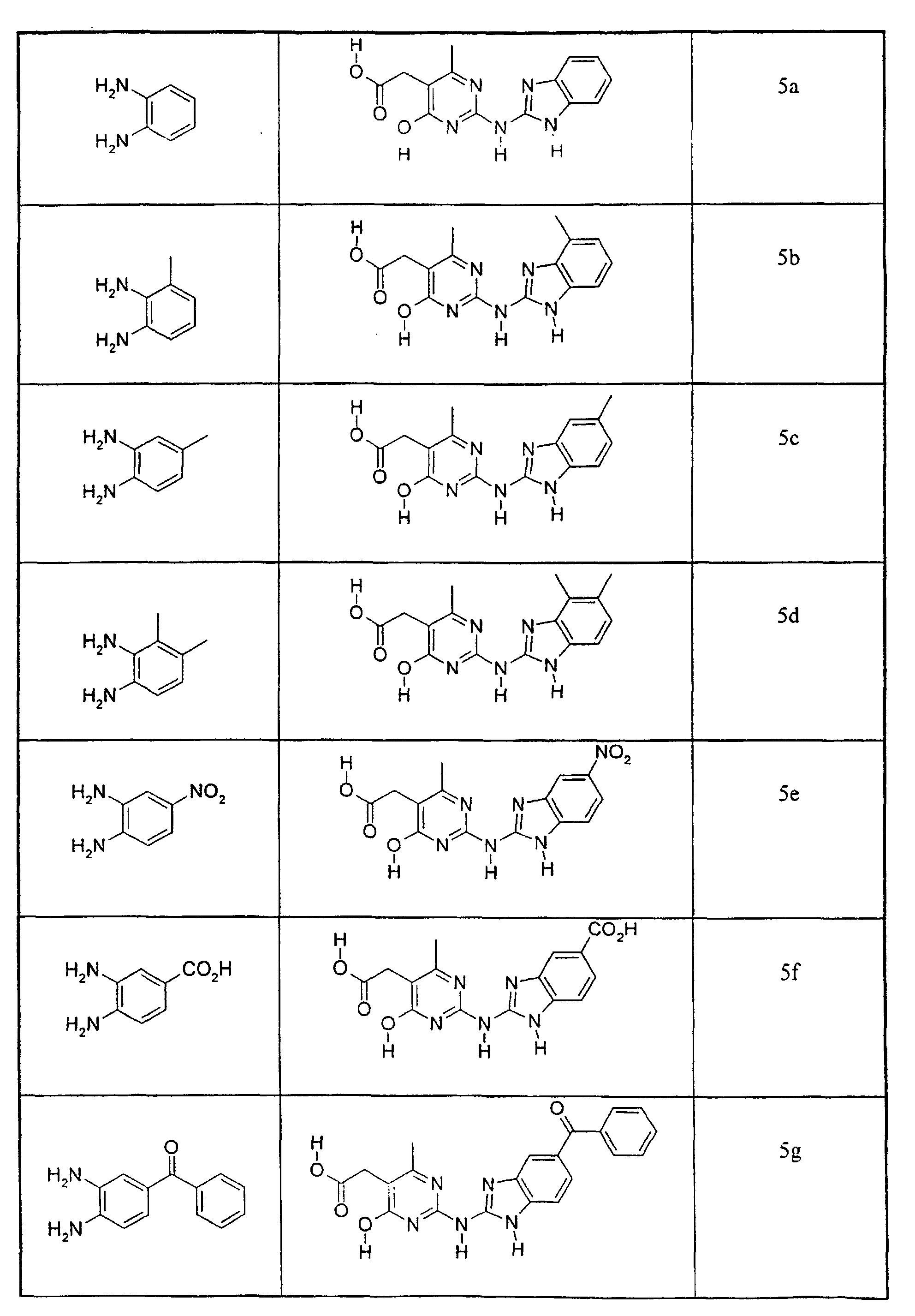

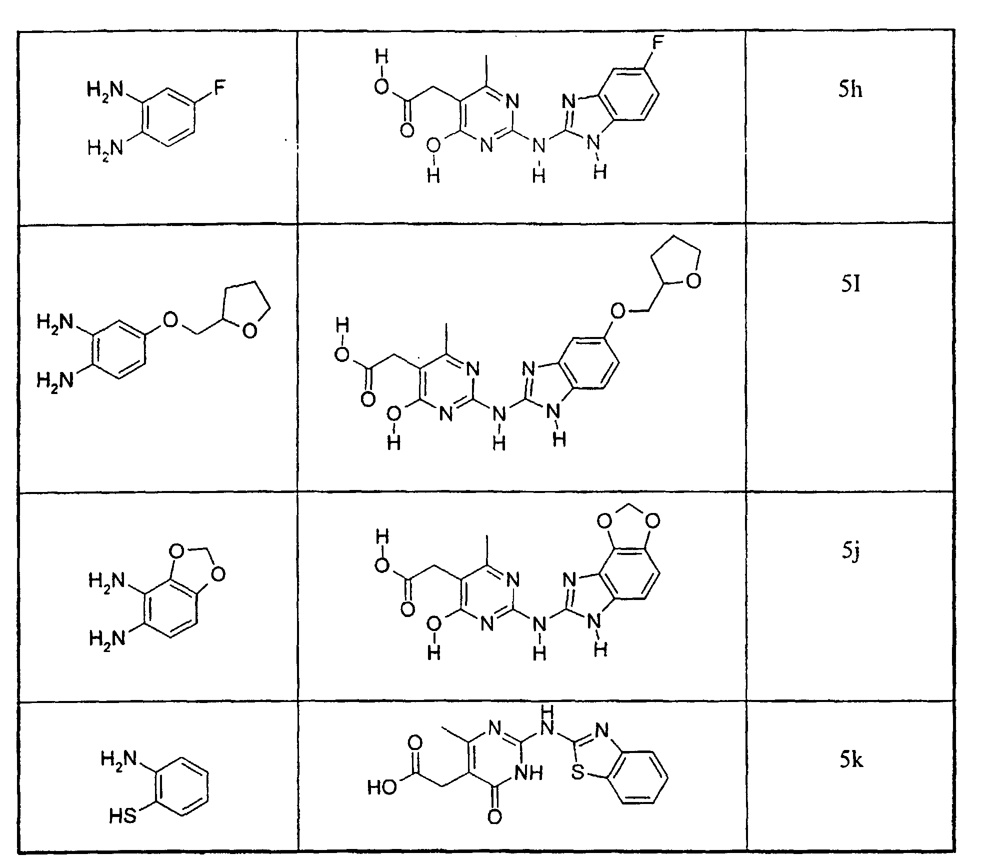

The reaction was carried in refluxing H₂O for 2 hours then cooled to room temperature and the precipitate was collected. Similarly benzothiazole analogues can be prepared. Thus 5k was prepared from the corresponding amino-thiophenol in 3M HCl at 100 °C/10hr, to give the product in 40% yield after recystallisation.

### 2) Via 2-guanidino-benzoimidazoles, scheme IV.

The parent acid (2) was obtained in 27% yield when 2-guanidino-5,6-dimethylimidazole 2-guanidino-5,6-dimethyl-benzimidazole, (6) was heated at 120 °C for 45min in neat diethyl acetylsuccinate. followed by refluxing in ethanol and recrytallisation from DMF.

In order to prepare a number of analogues of (2) by this route a series of 2-guanidinobenzoimidazoles were prepared (scheme V).

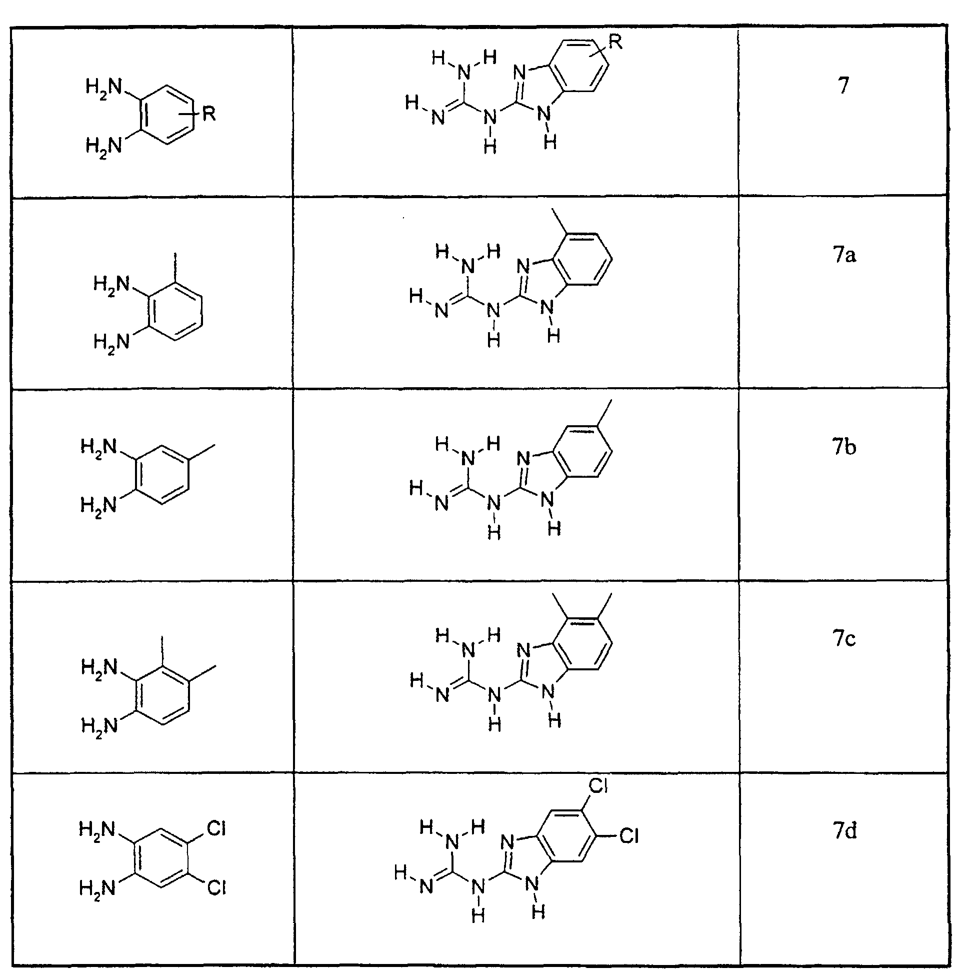

Similarly heating 2-guanidinobenzothiazole and ethyl acetoacetonate together at 110°C for 45 min gave (8)

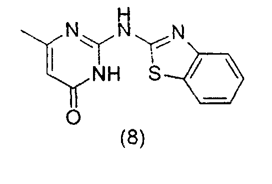

### Preparation of 6-Alkyl analogues of 2-[(5,6-Dimethyl-2-benzoimidazolyl)amino]-4-hydroxy-6-methyl-5-pyrimidine acetic acid (2)

These analogues were prepared in a similar fashion to (2)

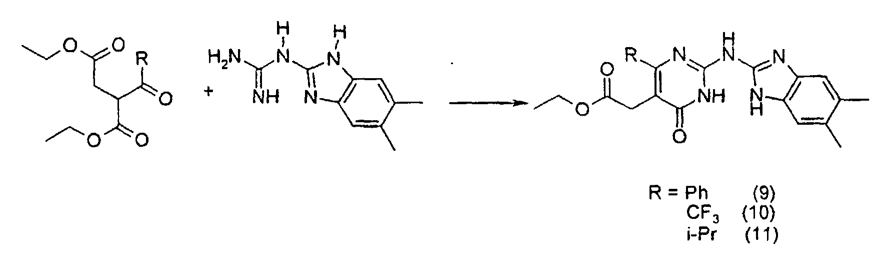

### Preparation of N-1 Alkyl Analogues of (2)

N-1 methyl analogues could be prepared in low yield during the methylation of the dithiocarbonimido using excess methyl iodide.

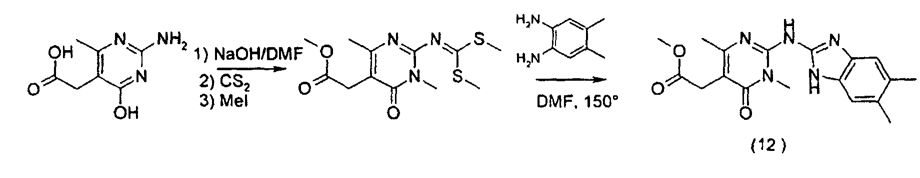

### EXAMPLES

### Example 1

### 2-[(5,6-Dimethyl-2-benzoimidazolyl)amino]-4-hydroxy-6-methyl-5-pyrimidine acetic acid (2)

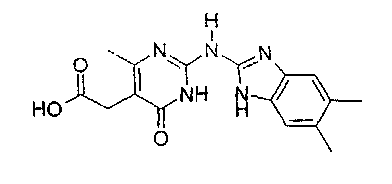

Under an inert atmosphere, Na (2-Cyanoamino)-4-hydroxy-6-methyl-5-pyrimidine acetic acid (4) (0.345 g. 15 mmol) was added portionwise to dry ethanol (10 mL) at room temperature. After stirring for 15 min (disapppearance of the Na), cyanoguanidine (0.840 g, 10 mmol) and diethyl acetylsuccinate (2.0 mL, 10 mmol) were added to the solution at room temperature.

The suspension was heated at reflux for 5h and the resulting suspension was cooled, filtered and washed with ethanol. The precipitate was dissolved in water (at the solubility limit) and concentrated HCl was added. The precipitate formed was filtered and gave 1.10 g (50%) of Na (2-Cyanoamino)-4-hydroxy-6-methyl-5-pyrimidine acetic acid. mp: 234-236 °C (dec). IR (KBr): υ = 3520, 3440, 3200-2500, 2190, 1730, 1655, 1602, 1500, 1340, 1200. 830 cm⁻¹.

(2-Cyanoamino)-4-hydroxy-6-methyl-5-pyrimidine acetic acid (4) (0.416 g, 2 mmol) and 5,6-dimethyl-1,2-diphenyldiamine (0.276 g, 2 mmol) in a mixture of H₂O (2 mL) and concentrated HCl (0.4 mL) were stirred for 1.5 h at 100 °C. The initial suspension dissolved and a second precipitate appeared after 1 h. The supension was cooled and the precipitate was collected and washed with water to give 0.164 g (25%) of 2-[(5,6-Dimethyl-2-benzoimidazolyl)amino]-4-hydroxy-6-methyl-5-pyrimidine acetic acid (2). mp: > 300 °C (DMF). IR (KBr): υ = 3331, 2949, 1705, 1655, 1602, 1509, 1252, 1157, 1047, 701 cm⁻¹. Accurate mass: calculated: 327.1331. Found 327.1337. Analysis calculated for C₁₆H₁₇N₅O₃: C, 58.71; H, 5.23; N, 21.39. Found: C, 57.8; H, 5.1; N, 20.7.

### Example 3

### 2-[(2-benzoimidazolyl)amino]-4-hydroxy-6-methyl-5-pyrimidine acetic acid (5a)

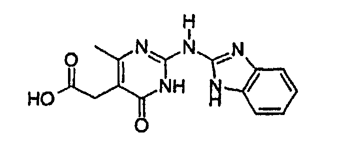

(5a) was prepared according to procedure used for (2) in Example 1; yield 5%. mp:> 300 °C (DMF). IR (KBr): υ = 2878, 1748, 1700, 1625, 1557, 1474, 1357, 1219, 744 cm⁻¹. ¹H-NMR (CF₃CO₂D): δ = 2.64 (s, 3H, CH₃), 3.88 (s, 2H, CH₂), 7.52 (m. 2H, 2 x Hₐᵣₒₘ), 7.67 (m, 2H, 2 x Hₐᵣₒₘ). Accurate mass: calculated: 299.1018. Found 299.1013. Analysis calculated for C₁₄H₁₃N₅O₃: C, 55.2; H, 4.40; N, 23.40. Found: C, 55.4: H, 4.7; N, 22.5.

### Example 4

### 2-[(4-methyl-2-benzoimidazolyl)amino]-4-hydroxy-6-methyl-5-pyrimidine acetic acid (5b)

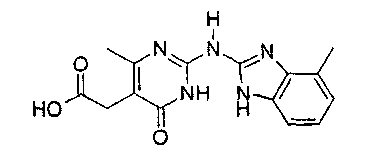

L-112787 was prepared according to procedure used for (2) in Example 1: yield 1%. mp: > 300 °C (DMF). IR (KBr): υ = 3150-2700. 1701, 1638, 1605, 1570, 1419, 1353, 1228, 781 cm⁻¹. Accurate mass: calculated: 313.1175. Found 313.1190. Analysis calculated for C₁₅H₁₅N₅O₃: C, 57.50; H, 4.83; N, 22.35. Found: C, 53.3; H, 4.5; N, 20.4.

### Example 5

### 2-[(5-methyl-2-benzoimidazolyl)amino]-4-hydroxy-6-methyl-5-pyrimidine acetic acid (5c)

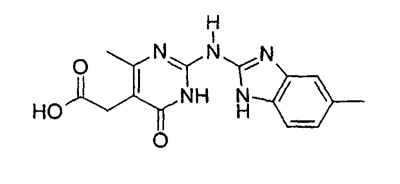

L-112788 was prepared according to procedure used for (2) in Example 1; yield 5%. mp: > 300 °C (DMF). IR (KBr): υ = 3200-2700, 1702, 1640, 1602, 1474, 1352, 1295, 1207, 875, 796 cm⁻¹. Accurate mass: calculated: 313.1175. Found 313.1187. Analysis calculated for C₁₅H₁₅N₅O₃: C, 57.50, H, 4.83, N, 22.35. Found: C, 53.3, H, 4.5, N, 20.4.

### Example 6

### 2-[(4,5-dimethyl-2-benzoimidazolyl)amino]-4-hydroxy-6-methyl-5-pyrimidine acetic acid (5d)

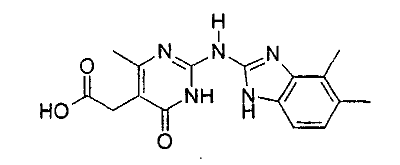

L-112789 was prepared according to procedure used for (2) in Example 1; yield 4%. mp: > 300 °C (DMF). IR (KBr): υ = 3200-2700. 1703, 1627, 1601, 1565, 1342, 1293, 1234, 903, 788 cm⁻¹. Accurate mass: calculated: 327.1331. Found 327.1349.

### Example 7

### 2-[(5-nitro-2-benzoimidazolyl)amino]-4-hydroxy-6-methyl-5-pyrimidine acetic acid (5e)

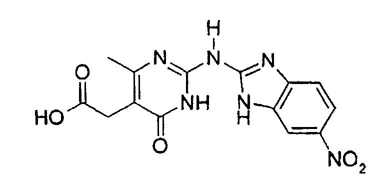

PNU-181223 was prepared according to procedure used for (2) in Example 1: yield 16%. mp: > 300 °C (DMF). IR (KBr): υ = 3039, 2882, 1738, 1596, 1465, 1333, 1292, 1182, 876 cm⁻¹. ¹H-NMR (CF₃CO₂D): δ = 2.62 (s, 3H, CH,), 3.87 (s, 2H, CH₂), 7.88 (d, 1H, *J* = 8 Hz, Hₐᵣₒₘ), 8.45 (d, 1H, *J* = 8 Hz, Hₐᵣₒₘ), 7.88 (s, 1H, Hₐᵣₒₘ). Analysis calculated for C₁₄H₁₂N₆O₅: C, 55.2; H, 4.40; N, 23.40. Found: C, 55.4; H, 4.7; N, 22.5.

### Example 8

### 2-[(5-fluoro-2-benzoimidazolyl)amino]-4-hydroxy-6-methyl-5-pyrimidine acetic acid (5h)

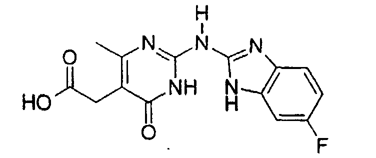

L-112790 was prepared according to procedure used for (2); yield 3%. mp:> 300 °C (DMF). IR (KBr): υ = 3150-2700, 1625, 1603, 1559, 1484, 1346, 1297, 1145, 790 cm⁻¹ . Accurate mass: calculated: 317.0924. Found 317.0939. Analysis calculated for C₁₄H₁₂FN₅O₃: C, 53.00; H, 3.81: N, 22.07. Found: C, 49.5; H, 4.0; N, 19.9.

### Example 9

### 2-(2'-Benzothiazolylamino)-4-hydroxy-6-methyl-5-pyrimidylacetic acid (5k)

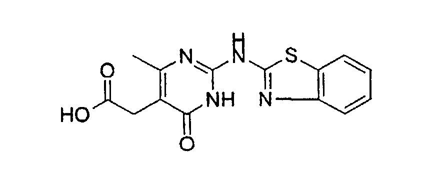

A mixture of 2-aminothiophenol (601.4 mg, 4.80 mmol) and 2-cyanamino-4-hydroxy-6-methyl-5-pyrimidylacetic acid (1.0 g 4.80 mmol) in concentrated HCl (1 mL) and water (5 mL) was stirred at 100 °C for 10 h. The reaction mixture was cooled and the precipitate was filtered and washed with aqueous ethanol. Crude yield 1.2 g (contains minor impurities according to ¹H NMR). The crude solid was recrystallized from DMF (∼ 150 mL). The product precipitated in the refrigerator. The crystalls were triturated with water/MeOH and dried under vaccum at 90 °C (yield 604.5 mg 40 %). mp > 300 °C. ¹H NMR (CF₃COD) d 7.97-7.86 (m, 2 H), 8.00-7.54 (m, 4 H), 3.90 (s, 3 H), 2.66 (s, 3 H); Anal. Calcd for C₁₄H₁₂N₄O₃S 0.25 H₂O: C. 51.67; H, 4.03; N, 17.22; Found: C, 51.80; H, 3.90; N, 16.95.

### Example 10

Benzimidazol-2-yl guanidino derivatives: General procedure for the intermediates In water (8 mL). concentrated HCI (3 mL). cyanoguanidine (20 mmol) and the *ortho*-phenylenediamine derivative (10 mmol) were heated at reflux overnight. A precipitate appeared after cooling, the solid was filtered and washed with water. A suspension of the solid in water, was treated with a solution of NaOH 1 M to reach pH 10. Then the basic solution was (pH 6) with an aqueous solution of HCI 1M and the resulting precipitate was filtered to give the title compound.

### Example 10a

### 2-Guanidino-4-methylbenzimidazole, hydrochloric salt (7a)

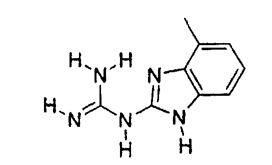

mp: > 260 °C IR (KBr): υ = 3500-2800, 1616, 1283, 1224, 1159, 963, 764, 729 cm⁻¹. Analysis calculated for C₉H₁₁N₅, HCl + 1/2 H₂O: C, 46.00, H, 5.5; N, 29.8. Found: C, 46.0; H, 5.7; N, 29.7.

### Example 10b

### 2-Guanidino-5-methylbenzimidazole, hydrochloric salt (7b)

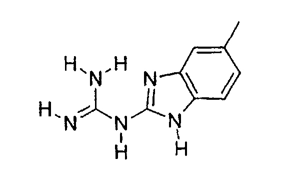

mp: > 260 °C IR (KBr): υ = 3500-2800, 1667, 1598, 1517, 1487, 1382, 1127, 1037, 802, 782, cm⁻¹. Analysis calculated for C₉H₁₅N₅, 1/2 HCl: C, 52.00; H, 5.54; N, 33.73. Found: C, 51.6; H, 5.5; N, 34.9.

### Example 10c

### 2-Guanidino-4,5-dimethylbenzimidazole, hydrochloric salt (7c)

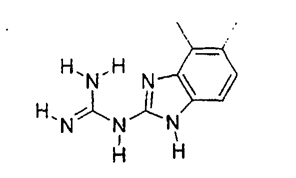

mp: > 260 °C IR (KBr): υ = 3500-2800, 3361, 1680, 1608, 1573, 1497, 1309, 1270, 1225, 1148, 1054, 791, 748, 723 cm⁻¹. Analysis calculated for C₁₀H₁₃N₅, HCl + 1/2 H₂O: C, 48.3; H, 6.0; N, 28.2. Found: C, 47.9; H, 5.9; N, 29.5.

### Example 10d

### 2-Guanidino-5,6-dichlorobenzimidazole, hydrochloric salt (7d)

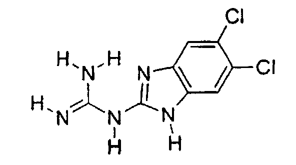

mp: > 260 °C IR (KBr): υ = 3623, 3496, 3500-2800, 1704, 1622, 1565, 1426, 1280, 1096, 862, 852 cm⁻¹. Analysis calculated for C₈H₇Cl₂N₅ HCl: C, 34.20; H, 2.85; N, 24.95. Found: C, 34.2; H, 3.3; N, 23.7.

### Example 10e

### 2-(2'-Benzothiazolylamino)-4-hydroxy-6-methylpyrimidine (8)

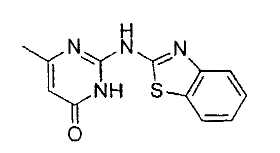

2-Guanidinobenzothiazole (0.384 g, 2 mmol) and ethyl acetoacetonate(2 mL) were heated together at 110°C for 45 min. After cooling, ethanol was added, and the resulting precipitate was filtrated to afford 0.050g (10%) of the tittle compound. mp:> 300°C. IR (KBr): υ = 3026, 2867, 1644, 1617, 1590, 1546, 1459, 1360, 762, 742, 640 cm⁻¹. Analysis calculated for C₁₂H₁₀N₄OS: C, 55.8, H, 3.90, N, 21.69. Found: C, 55.2, H, 4.2; N, 22.2.

### Example 11

### 2-[(5,6-Dimethyl-2-benzoimidazolyl)amino]-4-hydroxy-6-phenyl-5-pyrimidine acetic acid, (9)

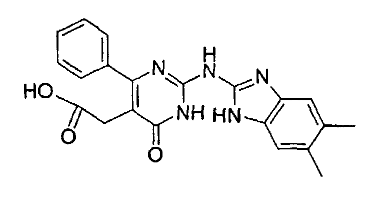

Diethyl-2-benzoyl succinate (2.70 g, 9.7 mmol) and 2-guanidino-5,6-dimethylbenzimidazole, PNU-10708A (6) (1.0 g, 4.17 mmol) was mixed with some EtOH to give a slurry. Sodium ethoxide (4.2 mL, 1 M) in ethanol was added and the solvent was evaporated. The mixture was heated at 120° C for 20 h and the reaction was followed by PDMS. The crude product was recrystallized from DMF. filtered and washed with acetone and water. During recrystallization the ester had been hydrolyzed and the product obtained was the corresponding acid. ¹H NMR (DMSO) δ 2.25 (s, 6H), 3.20 (s, 2H), 7.09 (s, 2H) and 7.53 (m. 5H).

### Example 12

### 2-[(5,6-Dimethyl-2-benzoimidazolyl)amino]-4-hydroxy-6-trifluoromethyl-5-pyrimidine acetic acid (10)

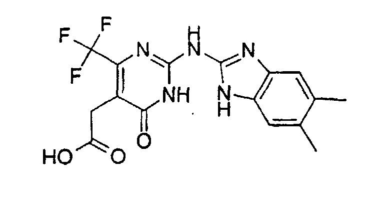

Diethyl-2-trifluoroacetyl succinate (0.40 g. 1.5 mmol) and 2-guanidino-5,6-dimethylbenzimidazole (6) ( 0.35 g, 1.5 mmol) was mixed with some EtOH to give a slurry. Sodium ethoxide (1.5 mL, 1 M) in ethanol was added and the solvent was evaporated. The mixture was heated at 100° C for 4 h. followed by 1 h heating at 150° C. The reaction was followed by PDMS. The crude product was recrystallized from DMF (5 mL), filtered and washed with acetone and water. During recrystallization the ester had been hydrolyzed and the product obtained was the corresponding acid. Gave 0.23 g as mixture of acid and ester. 50 mg was hydrolyzed in NaOH (50 mL, 1 M). The solution was then acidified and the precipitated product was collected to give 30 mg. ¹H NMR (DMSO) δ 2.27 (s, 6H), 3.43 (s, 2H) and 7.18 (s, 2H).

### Example 13

### 2-[(5,6-Dimethyl-2-benzoimidazolyl)amino]-4-hydroxy-6-isopropyl-5-pyrimidine acetic acid, (11)

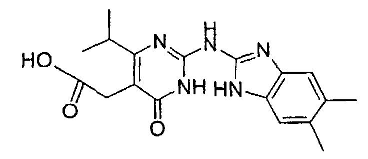

2-Guanidino-5,6-dimethyl-benzimidazole, (6) (1.24 g, 5.17 mmol) was dissolved in EtOH and ethoxide (5.2 mL, 1 M) in ethanol was added. The salt formed was filtered and diethyl-2-isobutyryl succinate (1.86 g, 7.6 mmol) was added and the solvent was followed by PDMS. The crude product was recrystallized from DMF, filtered and washed with acetone and water. The product was hydrolyzed in NaOH (50 mL, 1 M), acidified with HCl and the precipitate was collected. The product was recrystallized from DMSO (5 mL), filtered and washed with water and acetone to give 54 mg. ¹H NMR (DMSO) δ 1.19 (d, 6H), 2.23 (s, 6H), 3.01 (q, 1H), 3.33 (s, 2H) and 7.09 (s, 2H).

### Example 14

### Methyl-2-[(5,6-Dimethyl-2-benzoimidazolyl)amino]-1,4-dimethyl-6-oxo-5-pyrimidinyl acetate, (12)

### (i) 4-Hydroxy-6-methyl-5-pyrimidine acetic acid,

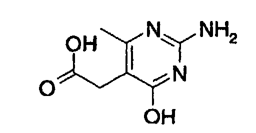

Guanidine hydrochloride (4.0 g, 42 mmol) was added to sodium ethoxide in ethanol (60 mL, 1 M) and the solution was stirred for 10 min. Diethylacetylsuccinate (9.0 g, 42 mmol) was added and the solution was refluxed for 4 h and then stirred over night at room temperature. Water (20 mL) was added to the mixture and the product was filtered and washed with water and EtOH. Yield: 4.2 g (55%). ¹H NMR (DMSO) δ 2.00 (s, 3H) and 3.22 (s, 2H).

### (ii) Methyl-2-[(di-(S)-methylthio)carbonimido]-N-methyl- 4-oxo-6-methyl-5-pyrimidine acetate

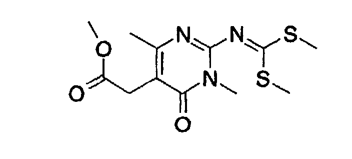

4-Hydroxy-6-methyl-5-pyrimidine acetic acid (1.0 g, *5.5* mmol) was mixed with DMF (150 mL). NaOH (6 mL, 20 M) was added and the mixture was allowed to cool and CS₂ (10 mL) was added. The dark red mixture was stirred for 30 min, followed by addition of MeI (10 mL). The colour changed to yellow and the mixture was stirred for 2 h. The reaction mixture was partitioned between water and methylene chloride and the organic phase was dried and evaporated. The crude product was purified on silica gel with a gradient of toluene:EtOAc (100% toluene-50% toluene). Yield:75 mg. (4%) ¹H NMR (DMSO) δ 2.18 (s, 3H), 2.58 (s, 6H), 3.41 (s, 3H), 3.50 (s, 2H) and 3.58 (s, 3H).

### (iii) Methyl-2- [(5,6-Dimethyl-2-benzoimidazolyl)amino]-1,4-dimethyl-6-oxo-5-pyrimidinyl acetate, (12)

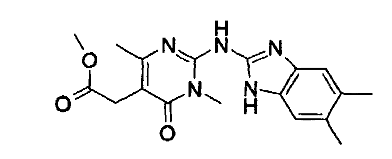

Methyl-2-[(di-(S)-methylthio)carbonimido]-N-methyl- 4-oxo-6-methyl-5-pyrimidine acetate (34 mg, 0.108 mmol) was dissolved in DMF (1 mL). A solution of 4,5-dimethyl-1,2-phenylenediamine (16 mg, 0.117 mmol) in a few drops of DMF was added and the solution was heated at 100 ° C for 1 h and at 150 ° C for 1 h more. The solution was allowed to cool and the solvent was evaporated. The product was purified on silica gel with chloroform : acetone: formic acid (gradient 96:3:1-89:10:1). Yield: 19 mg. (50%)
Mp: 272-274° C.

### Example 15

### 2-{4-methyl-6-oxo-2-[(1,5,6-trimethyl-1H-benzimidazo]-2-yl)amino-1,6-dihydro-5-pyrimidinyl} acetic acid

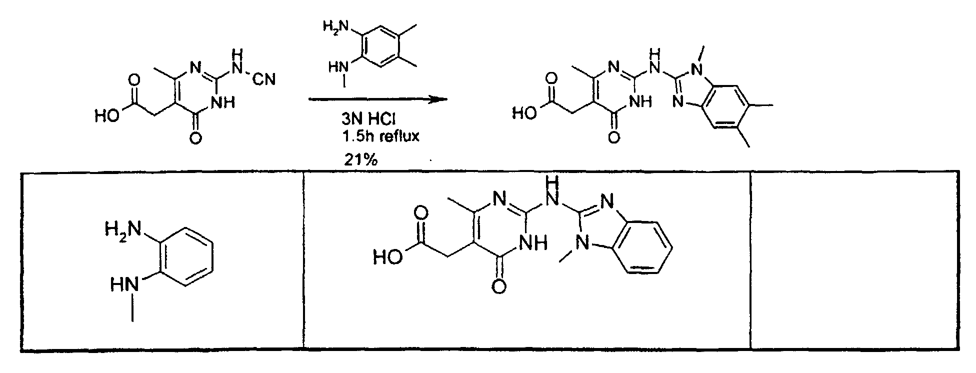

A suspension of *N*,4,5-trimethylphenylene-1,2-diamine (541 mg, 3.6 mmol) and (2-cyanoamino)-4-hydroxy-6-methyl-5-pyrimidineacetic acid (625 mg, 3.0 mmol) in 3*N* aqueous HCl was refluxed (bath 160° C) for 90 minutes. The heating was switched off and the reaction mixture was left to cool down over night. The white solid that had formed was isolated by filtration, washed with cold water and dried in vacuum over P₂O₅. The very insoluble off-white solid (213 mg, 21%) was suspended in a small amount of water and the pH was raised to 7.0 (from 2.0) by addition of an aqueous solution of NaOH. The mixture was concentrated and dried yielding a brownish solid. ¹H NMR (DMSO) δ 2.16 (s, 3H, CH3), 2.27 (s, 3H, CH3). 2.30 (s, 3H, CH3), 3.18 (s, 2H, CH2), 3.58 (s, 3H, N-CH3), 7.12 (s, 1H, CH), 7.26 (s. 1H, CH); ¹³C NMR (DMSO) δ 19.9, 28.0, 31.7, 109.1. 115.0, 129.1, 130.6, 153.3, 163.0, 173.0; MS (EI+, [M]⁺) m/z 341; Anal. Calcd. (found) for C₉H₁₄N₂ (incl. 1.7 eq. NaOH): C 50.00 (49.88) % H 5.00 (5.10) % N 17.11 (16.90)%.

### BIOLOGICAL ACTIVITY

### Example 16.

### Dimerisation assay

A slightly modified fluoresence quenching assay previously described by Cunningham BC *et al.,* 1991, Science 254, 821-825 was used.

Hormone induced receptor oligomerization via the formation of a 1:2 complex with the extracellular domain of its receptor or binding protein (hGHbp) has been identified as a first critical step in hGH signal transduction. The binding of hGH to recombinantly expressed hGHbp which has been labeled with a flourescent label such as rhodamine results in a diminished signal. A small modecule which mimics the quench induced upon hGH binding in this assay may also mimic the full biological effects of native hormone.

### Materials and Methods

The protein reagents used in this assay were recombinantly produced in *E.Coli.* These included the hGH, reference active, and a modified form of hGHbp. The modification was used to facilitate labeling and consisted of hGHbpGly₄Cys. This is hereinafter designated hGHbp1433. HGHbp1433 was subsequently labeled with rhodamine, hereinafter designated rho-hGHbp.

### Results

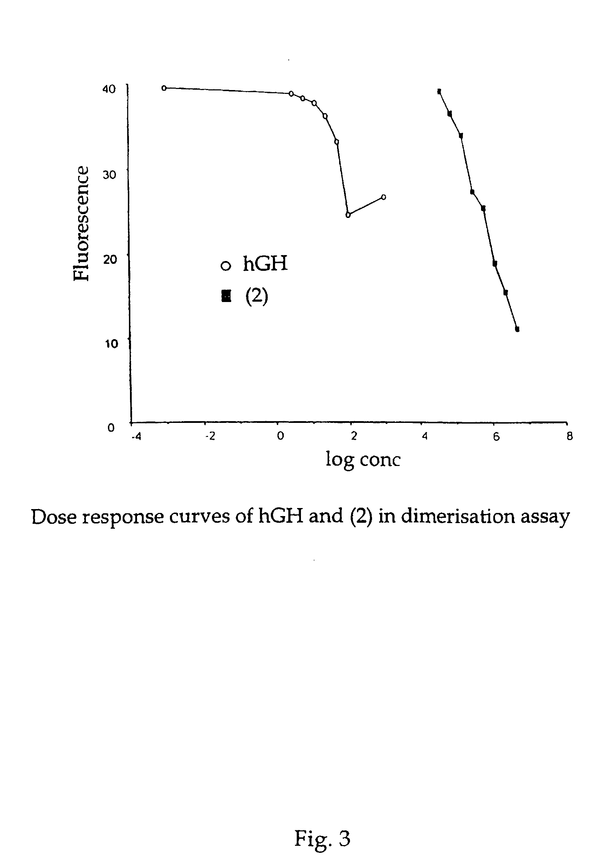

In Fig 3 the effects on fluorescence quenching in dimerisation assay is shown for hGH and (2). In both cases clear dose response curves could be detected in repeated experiments.

### Example 17.

### Glucose uptake.

### Materials and Methods

*Cell culture.*AT-1 cells from left atrial-derived transplantable tumors were established in primary cultures as earlier described by Steinhelper M.E *et al.,* Am. J. Physiol. 259, 1826-1834, 1990.

*Glucose transport.* Glucose transport was assayed as described by Hundal et al Biochem J. 297, 289-295, 1994.

Cells were kept in low glucose DMEM without serum for 5 hours prior hormone addition. After incubation with hormones for 60 minutes if not otherwise stated cell monolayers were rinsed with PBS. Glucose uptake was quantified by incubating the cells in the presence of 0.1 Ci/ml ³H-2-deoxy-glucose in PBS for 4 minutes. Non specific uptake was determined by quantifying cell associated radioactivity in the presence of 20 uM CytochalasinB. Uptake of ³H-2-deoxy-glucose was terminated by rapidly aspirating the medium followed by two successive washes with ice cold PBS. The cells were lysed in 0.5 M NaOH and followed by liquid scintillation counting. Rates of transport were normalized for protein content in each well.

*Materials.* Female B6D2/F1 mice were purchased from Bomholt Gård, Denmark. At-1 cells was provided by dr. W.Claycomb. Louisiana State University Medical Center, New Orleans, Louisiana.

Joklik's MEM, Dulbecko's MEM, Pen/Strep and Foetal Calf Serum (FCS) and all plastic ware were from Life Technologies. Collagenase was from Worthington biochemical Corp. and Trypsin (pig pancreas) from US Biochemical Corp. USA. ³H-2-deoxy-glucose was from Du Pont NEN. duMedical Scandinavia, Sweden. Human Growth Hormone, Genotropin batch 68199-51 was from Pharmacia & Upjohn, Sweden. CytochalasinB, bovine insulin and Wortmannin were from Sigma.

### Results.

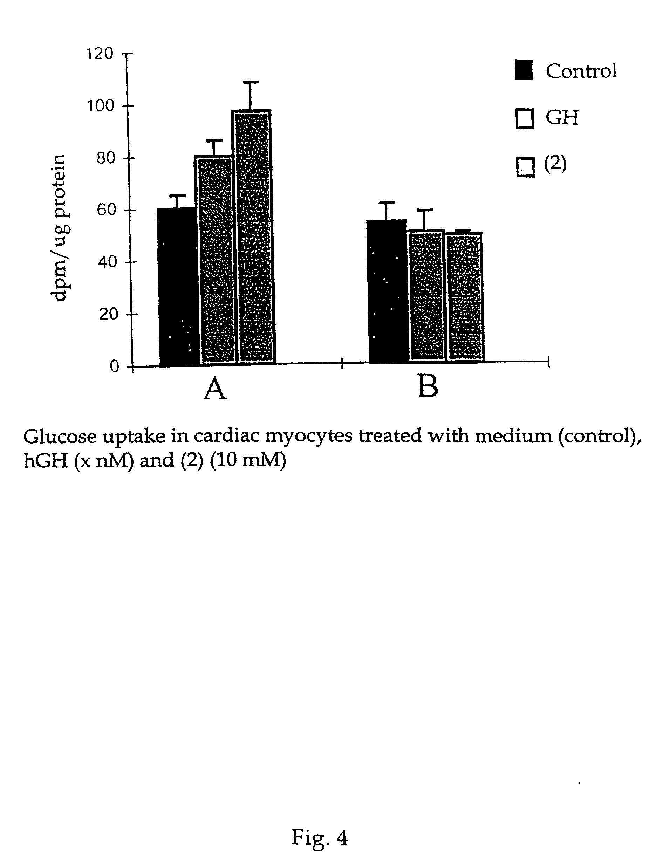

When 10 uM of the compound (2) was incubated for 1 hour prior glucose transport measurement the substance increased glucose transport to the same extent as GH (Fig4A). When cardiomyocytes were incubated with 1 uM Wortmannin together with hGH or the compound for 60 minutes prior transport measurements, the wortmannin treated cells did not respond with increased glucose transport (Fig 4B). This was due to an inhibition of a signal generated from the receptor by inhibiting the signal transducer phosphoinositide 3-kinase (PI 3-kinase).

## Claims

1. Compounds of the general formula (I) 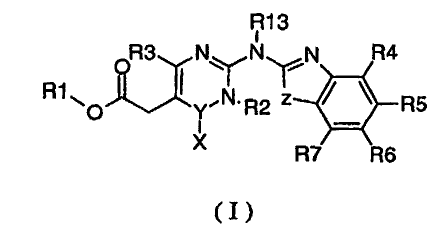 in which
Y-X is >C = X when X is NR8, O, or S and R2 is H, a straight or branched chain with 1 to 8 carbon atoms; a straight or branched chain substituted with a ring having 5-12 carbon atoms and optionally having one or two hetero atoms, a ring having 5-12 carbon atoms and optionally having one or two hetero atoms substituted with halogen, -OH, a straight or branched chain with 1 to 8 carbon atoms, OR10, NR10, R11, NO2, CF3, CN, COR8 and/or COOR8; or
Y-X is >C-X when X is H, a straight or branched chain with 1 to 8 carbon atoms, a straight or branched chain with 1 to 8 carbon atoms substituted with halogen, -OH, a straight or branched chain with 1 to 8 carbon atoms OR10, NR10, R11, NO2, CF3, CN, COR8 and/or COOR8, a ring having 5-12 carbon atoms and optionally having one or two hetero atoms, a ring having 5-12 carbon atoms and optionally having one or two hetero atoms substituted with halogen, -OH, a straight or branched chain with 1 to 8 carbon atoms, OR10, NR10, R11, NO2, CF3, CN, COR8 and/or COOR8, or X is OR9 or NHR9 and R2 is a bond to Y;
Z=O, S or NR12;
R1 is H, methyl, propyl, butyl or isoforms thereof, a straight or branched chain with 1 to 8 carbon atoms substituted with halogen, -OH, a straight or branched chain with 1 to 8 carbon atoms, OR10, NR10, R11, NO2, CF3, CN, COR8 and/or COOR8, a ring having 5-12 carbon atoms and optionally having one or two hetero atoms or a ring having 5-12 carbon atoms and optionally having one or two hetero atoms or a ring having 5-12 carbon atoms and optionally having one or two hetero atoms substituted with halogen, -OH, a straight or branched chain with 1 to 8 carbon atoms, OR10, NR10, R11, NO2, CF3, CN, COR8 and/or COOR8;
R3 is H, a straight or branched chain with 1 to 8 carbon atoms, a straight or branched chain with 1 to 8 carbon atoms substituted with halogen, -OH, a straight or branched chain with 1 to 8 carbon atoms OR10, NR10, R11, NO2, CF3, CN, COR8 and/or COOR8, a ring having 5-12 carbon atoms and optionally having one or two hetero atoms or a ring having 5-12 carbon atoms and optionally having one or two hetero atoms substituted with halogen, -OH, a straight or branched chain with 1 to 8 carbon atoms, OR10, NR10, R11, NO2, CF3, CN, COR8 and/or COOR8, or R³ is OR9 or NHR9;
R4, R5, R6, R7 are H, halogen, OR10, NR10, R11, NO2, CF3, CN, COR8, COOR8, CONHR8 and/or R3 in any combination and/or two adjacent R4, R5, R6 or R7 form a carbocyclic or heterocyclic ring;
R8, R9, R10, R11, R12 and R13 are H, a straight or branched chain with 1 to 8 carbon atoms, a straight or branched chain substituted with a ring having 5-12 carbon atoms and optionally having one or two hetero atoms, a ring having 5-12 carbon atoms and optionally having one or two hetero atoms and/or a ring having 5-12 carbon atoms and optionally having one or two hetero atoms substituted halogen, -OH, a straight or branched chain with 1 to 8 carbon atoms OR10, NR10, R11, NO2, CF3, CN, COR8 and/or COOR8 with in any combination.

2. Compound according to claim 1 selected from any of
2-[(5,6-Dimethyl-2-benzoimidazolyl)amino]-4-hydroxy-6-methyl-5-pyrimidine acetic acid (2)
2-[(5,6-Dimethyl-2-benzoimidazolyl)amino]-4-hydroxy-6-phenyl-5-pyrimidine acetic acid (9)
2-[(5,6-Dimethyl-2-benzoimidazolyl)amino]-4-hydroxy-6-trifluoromethyl-5-pyrimidine acetic acid (10)
Methyl-2-[(5,6-Dimethyl-2-benzoimidazolyl)amino]-1-4-dimethyl-6-oxo-5-pyrimidinyl acetate, (12)

3. Compounds according to any of claims 1 to 2 for use as an active therapeutic substance.

4. Compounds of the general formula (I) 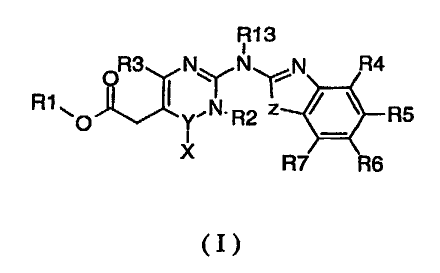 in which
Y -X is >C = X when X is NR8, O, or S and R2 is H, a straight or branched chain with 1 to 8 carbon atoms; a straight or branched chain substituted with a ring having 5-12 carbon atoms and optionally having one or two hetero atoms, a ring having 5-12 carbon atoms and optionally having one or two hetero atoms substituted with halogen, -OH, a straight or branched chain with 1 to 8 carbon atoms, OR10, NR10, R11, NO2, CF3, CN, COR8 and/or COOR8; or
Y-X is >C-X when X is a straight or branched chain with 1 to 8 carbon atoms, a straight or branched chain with 1 to 8 carbon atoms substituted with halogen, -OH, a straight or branched chain with 1 to 8 carbon atoms; OR10, NR10, R11, NO2, CF3, CN, COR8 and/or COOR8, a ring having 5-12 carbon atoms and optionally having one or two hetero atoms, a ring having 5-12 carbon atoms and optionally having one or two hetero atoms substituted with halogen, -OH, a straight or branched chain with 1 to 8 carbon atom, OR10, NR10, R11, NO2, CF3, CN, COR8 and/or COOR8, or X is OR9 or NHR9 and R2 is a bond to Y;
Z=O, S or NR12;
Z = O, S or NR12;
R1 is H, methy, propyl, butyl or isoforms thereof a straight or branched chain with 1 to 8 carbon atoms substituted with halogen, -OH, a straight or branched chain with 1 to 8 carbon atoms; OR10, NR10, R11, NO2, CF3, CN, COR8 and/or COOR8, a ring having 5-12 carbon atoms and optionally having one or two hetero atoms or a ring having 5-12 carbon atoms and optionally having one or two hetero atoms substituted with halogen, -OH, a straight or branched chain with 1 to 8 carbon atoms, OR10, NR10, R11, NO2, CF3, CN, COR8 and/or COOR8;
R3 is H, a straight or branched chain with 1 to 8 carbon atoms, a straight or branched chain with 1 to 8 carbon atoms substituted with halogen, -OH, a straight or branched chain with 1 to 8 carbon atoms; OR10, NR10, R11, NO2, CF3, CN, COR8 and/or COOR8, a ring having 5-12 carbon atoms and optionally having one or two hetero atoms or a ring having 5-12 carbon atoms and optionally having one or two hetero atoms substituted with halogen, -OH, a straight or branched chain with 1 to 8 carbon atoms; OR10, NR10, R11, NO2, CF3, CN, COR8 and/or COOR8, or R³ is OR9 or NHR9;
R4, R5, R6, R7 are H, halogen, OR10, NR10, R11, NO2, CF3, CN, COR8, COOR8, CONHR8 and/or R3 in any combination and/or two adjacent R4, R5, R6 or R7 form a carbocyclic or heterocyclic ring;
R8, R9, R10, R11, R12, R13 are H, a straight or branched chain with 1 to 8 carbon atoms, a straight or branched chain substituted with a ring having 5-12 carbon atoms and optionally having one or two hetero atoms, a ring having 5-12 carbon atoms and optionally having one or two hetero atoms and/or a ring having 5-12 carbon atoms and optionally having one or two hetero atoms substituted halogen, -OH, a straight or branched chain with 1 to 8 carbon atom, OR10, NR10, R11, NO2, CF3, CN, COR8 and/or COOR8 with in any combination
for use as an active therapeutic substance in the treatment of growth hormone deficiency, Turner syndrome, infertility, wound healing, dystrophy, osteoporosis and/or lactation failure.

5. Pharmaceutical composition comprising the compounds according to any of claims 1 to 4 with a pharmaceutically acceptable carrier

6. Method for the preparation of the compounds according to any of claims 1 to 4 by any of the following routes:
a) Reacting cyanoamine pyrimidine derivatives together with phenyldiamines or 2-amino thiophenols or 2-amino phenols or
b) Reacting 2-guanidino-benzoimidazoles together with diethyl acylsuccinate.

7. Process for the preparation of a composition according to claim 6, which comprises the mixing of the compound of the general formula I and a pharmaceutically acceptable carrier.

8. Use of compounds according to any of claims 1 to 4 for the preparation of medicaments for the treatment of growth hormone deficiency, Turner syndrome, infertility, wound healing, dystrophy, osteoporosis and/or lactation failure.

## Patentansprüche

1. Verbindungen der allgemeinen Formel (1): 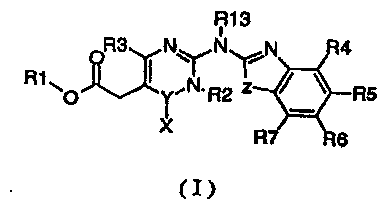 worin:
Y-X ist >C=X, wenn X NR8, O oder S ist und R2 H, eine gerade oder verzweigte Kette mit 1 bis 8 Kohlenstoffatomen; eine gerade oder verzweigte Kette, substituiert mit einem Ring mit 5 bis 12 Kohlenstoffatomen und gegebenenfalls mit einem oder zwei Heteroatomen, einem Ring mit 5 bis 12 Kohlenstoffatomen und gegebenenfalls einem oder zwei Heteroatomen, substituiert mit Halogen, -OH, einer geraden oder verzweigten Kette mit 1 bis 8 Kohlenstoffatomen, OR10, NR10, R11, NO2, CF3, CN, COR8 und/oder COOR8 ist; oder
Y-X ist >C-X, wenn X H, eine gerade oder verzweigte Kette mit 1 bis 8 Kohlenstoffatomen, eine gerade oder verzweigte Kette mit 1 bis 8 Kohlenstoffatomen, substituiert mit Halogen, -OH, einer geraden oder verzweigten Kette mit 1 bis 8 Kohlenstoffatomen; OR10, NR10, R11, NO2, CF3, CN, COR8 und/oder COOR8, ein Ring mit 5 bis 12 Kohlenstoffatomen und gegebenenfalls mit einem oder zwei Heteroatomen, ein Ring mit 5 bis 12 Kohlenstoffatomen und gegebenenfalls mit einem oder zwei Heteroatomen, substitiüert mit Halogen, -OH, einer geraden oder verzweigten Kette mit 1 bis 8 Kohlenstoffatomen, OR10, NR10, R11, NO2, CF3, CN, COR8 und/oder COOR8 ist, oder X OR9 oder NHR9 ist und R2 eine Bindung zu Y ist;
Z = O, S oder NR12;
R1 ist H, Methyl; Propyl, Butyl oder Isoformen davon, eine gerade oder verzweigte Kette mit 1 bis 8 Kohlenstoffatomen, substituiert mit Halogen, -OH, einer geraden oder verzweigten Kette mit 1 bis 8 Kohlenstoffatomen, OR10, NR10, R11, NO2, CF3, CN, COR8 und/oder COOR8, ein Ring mit 5 bis 12 Kohlenstoffatomen und gegebenenfalls mit einem oder zwei Heteroatomen, oder ein Ring mit 5 bis 12 Kohlenstoffatomen und gegebenenfalls mit einem oder zwei Heteroatomen, oder ein Ring mit 5 bis 12 Kohlenstoffatomen und gegebenenfalls mit einem oder zwei Heteroatomen, substituiert mit Halogen, -OH, einer geraden oder verzweigten Kette mit 1 bis 8 Kohlenstoffatomen, OR10, NR10, R11, NO2, CF3, CN, COR8 und/oder COOR8;
R3 ist H, eine gerade oder verzweigte Kette mit 1 bis 8 Kohlenstoffatomen, eine gerade oder verzweigte Kette mit 1 bis 8 Kohlenstoffatomen, substituiert mit Halogen, -OH, einer geraden oder verzweigten Kette mit 1 bis 8 Kohlenstoffatomen, OR10, NR10, R11, NO2, CF3, CN, COR8 und/oder COOR8, ein Ring mit 5 bis 12 Kohlenstoffatomen und gegebenenfalls mit einem oder zwei Heteroatomen oder ein Ring mit 5 bis 12 Kohlenstoffatomen und gegebenenfalls mit einem oder zwei Heteroatomen, substituiert mit Halogen, -OH, einer geraden oder verzweigten Kette mit 1 bis 8 Kohlenstoffatomen, OR10, NR10, R11, NO2, CF3, CN, COR8 und/oder COOR8, oder R3 ist OR9 oder NHR9;
R4, R5, R6, R7 sind H, Halogen, OR10, NR10, R11, NO2, CF3, CN, COR8, COOR8, CONHR8 und/oder R3 in jedweder Kombination, und/oder zwei benachbarte R4, R5, R6 oder R7 bildeneinen carbocyclischen oder heterocyclischen Ring;
R8, R9, R10, R11, R12 und R13 sind H, eine gerade oder verzweigte Kette mit 1 bis 8 Kohlenstoffatomen, eine gerade oder verzweigte Kette, substituiert mit einem Ring mit 5 bis 12 Kohlenstoffatomen und gegebenenfalls mit einem oder zwei Heteroatomen, einem Ring mit 5 bis 12 Kohlenstoffatomen und gegebenenfalls mit einem oder zwei Heteroatomen und/oder einem Ring mit 5 bis 12 Kohlenstoffatomen und gegebenenfalls mit einem oder zwei Heteroatomen, substituiert mit Halogen, -OH, einer geraden oder verzweigten Kette mit 1 bis 8 Kohlenstoffatomen; OR10, NR10, R11, NO2, CF3, CN, COR8 und/oder COOR8 in einer beliebigen Kombination.

2. Verbindung gemäß Anspruch 1, gewählt aus beliebigen von
2-[(5,6-Dimethyl-2-benzoimidazolyl)amino]-4-hydroxy-6-methyl-5-pyrimidinessigsäure (2)
2-[(5,6-Dimethyl-2-benzoimidazolyl)amino]-4-hydroxy-6-phenyl-5-pyrimidinessigsäure (9)
2-[(5,6-Dimethyl-2-benzoimidazolyl)amino]-4-hydroxy-6-trifluormethyl-5-pyrimidinessigsäure (10)
Methyl-2-[(5,6-dimethyl-2-benzoimidazolyl)amino]-1,4-dimethyl-6-oxo-5-pyrimidinylacetat (12)

3. Verbindungen gemäß mindestens einem der Ansprüche 1 bis 2 zur Verwendung als eine aktive therapeutische Substanz.

4. Verbindungen der allgemeinen Formel (1): 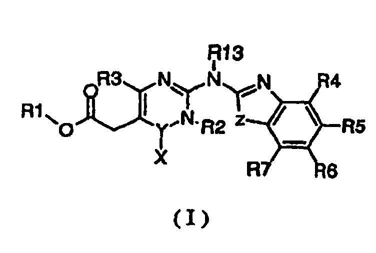 worin:
Y-X ist >C=X, wenn X NR8, O oder S ist und R2 H, eine gerade oder verzweigte Kette mit 1 bis 8 Kohlenstoffatomen; eine gerade oder verzweigte Kette, substituiert mit einem Ring mit 5 bis 12 Kohlenstoffatomen und gegebenenfalls mit einem oder zwei Heteroatomen, einem Ring mit 5 bis 12 Kohlenstoffatomen und gegebenenfalls einem oder zwei Heteroatomen, substituiert mit Halogen, -OH, einer geraden oder verzweigten Kette mit 1 bis 8 Kohlenstoffatomen, OR10, NR10, R11, NO2, CF3, CN, COR8 und/oder COOR8 ist; oder
Y-X ist >C-X, wenn X H, eine gerade oder verzweigte Kette mit 1 bis 8 Kohlenstoffatomen, eine gerade oder verzweigte Kette mit 1 bis 8 Kohlenstoffatomen, substituiert mit Halogen, -OH, einer geraden oder verzweigten Kette mit 1 bis 8 Kohlenstoffatomen; OR10, NR10, R11, NO2, CF3, CN, COR8 und/oder COOR8, ein Ring mit 5 bis 12 Kohlenstoffatomen und gegebenenfalls mit einem oder zwei Heteroatomen, ein Ring mit 5 bis 12 Kohlenstoffatomen und gegebenenfalls mit einem oder zwei Heteroatomen, substituiert mit Halogen, -OH, einer geraden oder verzweigten Kette mit 1 bis 8 Kohlenstoffatomen, OR10, NR10, R11, NO2, CF3, CN, COR8 und/oder COOR8 ist, oder X OR9 oder NHR9 ist und R2 eine Bindung zu Y ist;
Z = O, S oder NR12;
R1 ist H, Methyl, Propyl, Butyl oder Isoformen davon, eine gerade oder verzweigte Kette mit 1 bis 8 Kohlenstoffatomen, substituiert mit Halogen, -OH, einer geraden oder verzweigten Kette mit 1 bis 8 Kohlenstoffatomen; OR10, NR10, R11, NO2, CF3, CN, COR8 und/oder COOR8, ein Ring mit 5 bis 12 Kohlenstoffatomen und gegebenenfalls mit einem oder zwei Heteroatomen, oder ein Ring mit 5 bis 12 Kohlenstoffatomen und gegebenenfalls mit einem oder zwei Heteroatomen, substituiert mit Halogen, -OH, einer geraden oder verzweigten Kette mit 1 bis 8 Kohlenstoffatomen, OR10, NR10, R11, NO2, CF3, CN, COR8 und/oder COOR8;
R3 ist H, eine gerade oder verzweigte Kette mit 1 bis 8 Kohlenstoffatomen, eine gerade oder verzweigte Kette mit 1 bis 8 Kohlenstoffatomen, substituiert mit Halogen, -OH, einer geraden oder verzweigten Kette mit 1 bis 8 Kohlenstoffatomen; OR10, NR10, R11, NO2, CF3, CN, COR8 und/oder COOR8, ein Ring mit 5 bis 12 Kohlenstoffatomen und gegebenenfalls mit einem oder zwei Heteroatomen oder ein Ring mit 5 bis 12 Kohlenstoffatomen und gegebenenfalls mit einem oder zwei Heteroatomen, substituiert mit Halogen, -OH, einer geraden oder verzweigten Kette mit 1 bis 8 Kohlenstoffatomen; OR10, NR10, R11, NO2, CF3, CN, COR8 und/oder COOR8, oder R3 ist OR9 oder NHR9;
R4, R5, R6, R7 sind H, Halogen, OR10, NR10, R11, NO2, CF3, CN, COR8, COOR8, CONHR8 und/oder R3 in jedweder Kombination, und/oder zwei benachbarte R4, R5, R6 oder R7 bilden einen carbocyclischen oder heterocyclischen Ring;
R8, R9, R10, R11, R12 und R13 sind H, eine gerade oder verzweigte Kette mit 1 bis 8 Kohlenstoffatomen, eine gerade oder verzweigte Kette, substituiert mit einem Ring mit 5 bis 12 Kohlenstoffatomen und gegebenenfalls mit einem oder zwei Heteroatomen, einem Ring mit 5 bis 12 Kohlenstoffatomen und gegebenenfalls mit einem oder zwei Heteroatomen und/oder einem Ring mit 5 bis 12 Kohlenstoffatomen und gegebenenfalls mit einem oder zwei Heteroatomen, substituiert mit Halogen, -OH, einer geraden oder verzweigten Kette mit 1 bis 8 Kohlenstoffatomen, OR10, NR10, R11, NO2, CF3, CN, COR8 und/oder COOR8 in einer beliebigen Kombination.
zur Verwendung als eine aktive therapeutische Substanz bei der Behandlung von Wachstumshormionmangel, Turner-Syndrom, Unfruchtbarkeit, Wundheilung, Dystrophie, Osteoporose und/oder Lactationsstörung.

5. Pharmazeutische Zusammensetzung, umfassend die Verbindung gemäß mindestens einem der Ansprüche 1 bis 4 mit einem pharmazeutisch annehmbaren Träger.

6. Verfahren zur Herstellung der Verbindungen gemäß mindestens einem der Ansprüche 1 bis 4 durch einen beliebigen der folgenden Wege:
a) Umsetzen von Cyanoaminpyrimidin-Derivaten zusammen mit Phenyldiaminen oder 2-Aminothiophenolen oder 2-Aminophenolen oder
b) Umsetzen von 2-Guanidino-benzoimidazolen zusammen mit Diethylacylsuccinat.

7. Verfahren zur Herstellung einer Zusammensetzung gemäß Anspruch 6, welches das Mischen der Verbindung der allgemeinen Formel I und eines pharmazeutisch annehmbaren Trägers umfasst.

8. Verwendung von Verbindungen gemäß irgendeinem der Ansprüche 1 bis 4 zur Herstellung von Medikamenten zur Behandlung von Wachstumshormonmangel, Turner-Syndrom, Unfruchtbarkeit, Wundheilung, Dystrophie, Osteoporose und/oder Lactationsstörung.

## Revendications

1. Composés de formule générale (I) 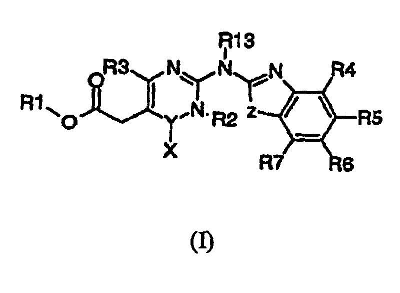 dans laquelle
Y-X est >C=X lorsque X est NR8, O ou S et R2 est H, une chaîne linéaire ou ramifiée avec 1 à 8 atomes de carbone ; une chaîne linéaire ou ramifiée substituée par un cycle possédant 5 à 12 atomes de carbone et ayant éventuellement un ou deux hétéroatomes, un cycle possédant 5 à 12 atomes de carbone et éventuellement un ou deux hétéroatomes substitué par un halogène, -OH, une chaîne linéaire ou ramifiée avec 1 à 8 atomes de carbone, OR10, NR10, R11, NO₂, CF3, CN, COR8 et/ou COOR8 ; ou
Y-X est >C-X lorsque X est H, une chaîne linéaire ou ramifiée avec 1 à 8 atomes de carbone, une chaîne linéaire ou ramifiée avec 1 à 8 atomes de carbone substituée par un halogène, -OH, une chaîne linéaire ou ramifiée avec 1 à 8 atomes de carbone, OR10, NR10, R11, NO2, CF3, CN, COR8 et/ou COOR8, un cycle possédant 5 à 12 atomes de carbone et éventuellement un ou deux hétéroatomes, un cycle possédant 5 à 12 atomes de carbone et éventuellement un ou deux hétéroatomes substitué par un halogène, -OH, une chaîne linéaire ou ramifiée avec 1 à 8 atomes de carbone, OR10, NR10, R11, NO2, CF3, CN, COR8 et/ou COOR8, ou X est OR9 ou NHR9 et R2 est une liaison avec Y ;
Z=O, S ou NR12;
R1 est H, un groupement méthyle, propyle, butyle ou leurs isoformes, une chaîne linéaire ou ramifiée avec 1 à 8 atomes de carbone substituée par un halogéne, -OH, une chaîne linéaire ou ramifiée avec 1 à 8 atomes de carbone, OR10, NR10, R11, NO2, CF3, CN, COR8 et/ou COOR8, un cycle possédant 5 à 12 atomes de carbone et éventuellement un ou deux hétéroatomes ou un cycle possédant 5 à 12 atomes de carbone et éventuellement un ou deux hétéroatomes substitué par un halogène, -OH, une chaîne linéaire ou ramifiée avec 1 à 8 atomes de carbone, OR10, NR10, R11, NO2, CF3, CN, COR8 et/ou COOR8;
R3 est H, une chaîne linéaire ou ramifiée avec 1 à 8 atomes de carbone, une chaîne linéaire ou ramifiée avec 1 à 8 atomes de carbone substituée par un halogène, -OH, une chaîne linéaire ou ramifiée avec 1 à 8 atomes de carbone, OR10, NR10, R11, NO2, CF3, CN, COR8 et/ou COOR8, un cycle possédant 5 à 12 atomes de carbone et éventuellement un ou deux hétéroatomes ou un cycle possédant 5 à 12 atomes de carbone et éventuellement un ou deux hétéroatomes substitué par un halogène, -OH, une chaîne linéaire ou ramifiée avec 1 à 8 atomes de carbone, OR10, NR10, R11, NO2, CF3, CN, COR8 et/ou COOR8, ou R3 est OR9 ou NHR9 ;
R4, R5, R6, R7 sont H, un halogène, OR10, NR10, R11, NO2, CF3, CN, COR8, COOR8, CONHR8 et/ou R3 dans une quelconque combinaison et/ou deux R4, R5, R6 ou R7 adjacents forment un cycle carbocyclique ou hétérocyclique ;
R8, R9, R10, R11, R12 et R13 sont H, une chaîne linéaire ou ramifiée avec 1 à 8 atomes de carbone, une chaîne linéaire ou ramifiée substituée par un cycle possédant 5 à 12 atomes de carbone et ayant éventuellement un ou deux hétéroatomes, un cycle possédant 5 à 12 atomes de carbone et éventuellement un ou deux hétéroatomes et/ou un cycle possédant 5 à 12 atomes de carbone et éventuellement un ou deux hétéroatomes substitué par un halogène, -OH, une chaîne linéaire ou ramifiée avec 1 à 8 atomes de carbone, OR10, NR10, R11, NO₂, CF3, CN, COR8 et/ou COOR8 dans une quelconque combinaison.

2. Composé selon la revendication 1, sélectionné parmi
Acide 2-[(5,6-diméthyl-2-benzoimidazolyl)amino]-4-hydroxy-6-méthyl-5-pyrimidine acétique (2)
Acide 2-[(5,6-diméthyl-2-benzoimidazolyl)amino]-4-hydroxy-6-phényl-5-pyrimidine acétique (9)
Acide 2-[(5,6-diméthyl-2-benzoimidazolyl)amino]-4-hydroxy-6-trifluorométhyl-5-pyrimidine acétique (10)
Acétate de méthyl-2-[(5,6-diméthyl-2-benzoimidazolyl)amino]-1,4-diméthyl-6-oxo-5-pyrimidinyle (12).

3. Composés selon l'une quelconque des revendications 1 à 2, pour utilisation comme substance thérapeutique active.

4. Composés de formule générale (I) 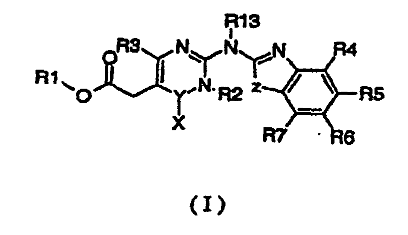 dans laquelle
Y-X est >C=X lorsque X est NR8, O ou S et R2 est H, une chaîne linéaire ou ramifiée avec 1 à 8 atomes de carbone ; une chaîne linéaire ou ramifiée substituée par un cycle possédant 5 à 12 atomes de carbone et ayant éventuellement un ou deux hétéroatomes, un cycle possédant 5 à 12 atomes de carbone et éventuellement un ou deux hétéroatomes substitué par un halogène, -OH, une chaîne linéaire ou ramifiée avec 1 à 8 atomes de carbone, OR10, NR10, R11, NO2, CF3, CN, COR8 et/ou COOR8 ; ou
Y-X est >C-X lorsque X est une chaîne linéaire ou ramifiée avec 1 à 8 atomes de carbone, une chaîne linéaire ou ramifiée avec 1 à 8 atomes de carbone substituée par un halogène, -OH, une chaîne linéaire ou ramifiée avec 1 à 8 atomes de carbone, OR10, NR10, R11, NO2, CF3, CN, COR8 et/ou COOR8, un cycle possédant 5 à 12 atomes de carbone et éventuellement un ou deux hétéroatomes, un cycle possédant 5 à 12 atomes de carbone et éventuellement un ou deux hétéroatomes substitué par un halogène, -OH, une chaîne linéaire ou ramifiée avec 1 à 8 atomes de carbone, OR10, NR10, R11, NO2, CF3, CN, COR8 et/ou COOR8, ou X est OR9 ou NHR9 et R2 est une liaison avec Y ;
Z = O, S ou NR12 ;
R1 est H, un groupement méthyle, propyle, butyle ou leurs isoformes, une chaîne linéaire ou ramifiée avec 1 à 8 atomes de carbone substituée par un halogène, -OH, une chaîne linéaire ou ramifiée avec 1 à 8 atomes de carbone, OR10, NR10, R11, NO2, CF3, CN, COR8 et/ou COOR8, un cycle possédant 5 à 12 atomes de carbone et éventuellement un ou deux hétéroatomes ou un cycle possédant 5 à 12 atomes de carbone et éventuellement un ou deux hétéroatomes substitué par un halogène, -OH, une chaîne linéaire ou ramifiée avec 1 à 8 atomes de carbone, OR10, NR10, R11, NO2, CF3, CN, COR8 et/ou COOR8;
R3 est H, une chaîne linéaire ou ramifiée avec 1 à 8 atomes de carbone, une chaîne linéaire ou ramifiée avec 1 à 8 atomes de carbone substituée par un halogène, -OH, une chaîne linéaire ou ramifiée avec 1 à 8 atomes de carbone, OR10, NR10, R11, NO2, CF3, CN, COR8 et/ou COOR8, un cycle possédant 5 à 12 atomes de carbone et éventuellement un ou deux hétéroatomes ou un cycle possédant 5 à 12 atomes de carbone et éventuellement un ou deux hétéroatomes substitué par un halogène, -OH, une chaîne linéaire ou ramifiée avec 1 à 8 atomes de carbone, OR10, NR10, R11, NO2, CF3, CN, COR8 et/ou COOR8, ou R3 est OR9 ou NHR9 ;
R4, R5, R6, R7 sont H, un halogène, OR10, NR10, R11, NO2, CF3, CN, COR8, COOR8, CONHR8 et/ou R3 dans une quelconque combinaison et/ou deux R4, R5, R6 ou R7 adjacents forment un cycle carbocyclique ou hétérocyclique ;
R8, R9, R10, R11, R12 et R13 sont H, une chaîne linéaire ou ramifiée avec 1 à 8 atomes de carbone, une chaîne linéaire ou ramifiée substituée par un cycle possédant 5 à 12 atomes de carbone et ayant éventuellement un ou deux hétéroatomes, un cycle possédant 5 à 12 atomes de carbone et éventuellement un ou deux hétéroatomes et/ou un cycle possédant 5 à 12 atomes de carbone et éventuellement un ou deux hétéroatomes substitué par un halogène, -OH, une chaîne linéaire ou ramifiée avec 1 à 8 atomes de carbone, OR10, NR10, R11, NO2, CF3, CN, COR8 et/ou COOR8 dans une quelconque combinaison ;
pour utilisation comme substance thérapeutique active dans le traitement du déficit en hormone de croissance, du syndrome de Turner, de la stérilité, de la cicatrisation des plaies, de la dystrophie, de l'ostéoporose et/ou du défaut de lactation.

5. Composition pharmaceutique comprenant les composés selon l'une quelconque des revendications 1 à 4, avec un véhicule pharmaceutiquement acceptable.

6. Procédé de préparation des composés selon l'une quelconque des revendications 1 à 4, par l'une quelconque des voies suivantes :
a) Réaction de dérivés de la cyanoamine pyrimidine avec des phényldiamines ou 2-amino thiophénols ou 2-amino phénols ou
b) Réaction de 2-guanidino-benzoimidazoles avec l'acylsuccinate de diéthyle.

7. Procédé de préparation d'une composition selon la revendication 6, qui comprend le mélange du composé de formule générale I et d'un véhicule pharmaceutiquement acceptable.

8. Utilisation de composés selon l'une quelconque des revendications 1 à 4 pour la préparation de médicaments destinés au traitement du déficit en hormone de croissance, du syndrome de Turner, de la stérilité, de la cicatrisation des plaies, de la dystrophie, de l'ostéoporose et/ou du défaut de lactation.
